# EUROPEAN PATENT SPECIFICATION

(11) **EP 3 412 266 B1**
(45) Date of publication and mention of the grant of the patent: **15.01.2020**
(21) Application number: 17175023.5
(22) Date of filing: 08.06.2017
(51) Int. Cl.: A61F 13/513

(54) **METHOD FOR VISUALIZING HORIZONTAL AIR PERMEABILITY**
VERFAHREN ZUR VISUALISIERUNG VON HORIZONTALER LUFTDURCHLÄSSIGKEIT
PROCÉDÉ DE VISUALISATION DE PERMÉABILITÉ À L'AIR HORIZONTALE

(43) Date of publication of application: 12.12.2018
(73) Proprietor: The Procter & Gamble Company, Cincinnati, OH 45202 (US)
(72) Inventor: Meignen Martinez, Paul Jesus, 13290-000 Louveira - Sao Paulo (BR); Quintero Ruiz, Mariana, 13290-000 Louveira - Sao Paulo (BR); Gonzalez Pino, Carlos Augusto, 13290-000 Louveira - Sao Paulo (BR)
(74) Representative: P&G Patent Germany

(56) References cited:
- EP-A2- 1 283 028
- WO-A2-2004/043317

## Description

### FIELD OF THE INVENTION

The present invention is directed at a method for assessing the horizontal air permeability of personal hygiene articles such as diapers. Horizontal air permeability relates to the ease at which an airflow can move on the wearer-facing surface of a personal absorbent hygiene article, rather than through the article (vertical air permeability). The method can be used to compare the horizontal air permeability of different articles in a simple manner.

### BACKGROUND OF THE INVENTION

Absorbent articles for personal hygiene such as diapers are designed to absorb and contain body exudates, in particular large quantity of urine. These absorbent articles comprise several layers, including a wearer-facing topsheet, a garment-facing backsheet and in-between an absorbent core. The topsheet contacts the skin of the wearer and should be soft and fluid-permeable, whereas the backsheet protects the environment of the wearer such as its clothes and should be impermeable to fluid. The absorbent core absorbs and retains the exudates for a prolonged amount of time to minimize re-wet and keep the wearer dry. An acquisition layer and/or a distribution layer may also be disposed between the topsheet and the absorbent core to facilitate the acquisition and the distribution of fluid inside the article.

Many modern diapers have excellent fluid acquisition and retention properties, so that they can be worn overnight without the need for changing the diaper. This means that the diaper will typically comprise a large amount of urine for a prolonged of time. It is however desirable to avoid excess humidity on the skin of the user. The skin of the wearer should feel as dry possible even after prolonged wearing of a wet diaper. Breathable backsheet films that allow humidity to escape the diapers have been commonly used in marketed diapers. These backsheet films have microholes that let vapor out while being impermeable to fluid. The breathability of these films is typically characterized by their water vapor transmission rate through the layer, expressed in grams per square meters per unit of time, as measured under standard conditions. This can be described as "vertical" or "through" permeability of the layer. However the amount of urine that is absorbed in the diaper is typically much higher that of what the breathable film can breathe out of the diaper during the average wearing time of the diaper. Since it is the humidity on the skin that is ultimately most relevant for the skin health, the horizontal air permeability of the topsheet may be considered to be at least as important to skin health as the vertical permeability of the backsheet. Horizontal air permeability, which may be also designated to as surface permeability, measures how easily an air flow can circulate in the plane of the wearer-facing side of the diaper rather than perpendicular to this plane.

US7,534,928B2 (Sakamoto et al.) discloses a method for measuring the horizontal air permeability of a small sample of a topsheet under a selected pressure. This method is indicated to provide relatively precise value. However it measures the topsheet taken in isolation of the rest of the article and independently of the presence of a fluid absorbed in the article. This laboratory method also requires a specific equipment which is costly to acquire if only a small number of measurements are desired.

The present invention addresses the problem of providing a simple, quick and cheap method for visualizing the horizontal air permeability of a diaper under simulated normal usage conditions.

### SUMMARY OF THE INVENTION

The method of the invention allows making a visual assessment of the horizontal air permeability on a region of interest of the wearer-facing side of a personal hygiene article, such as a diaper. The method comprising the subsequent steps of:
- providing an absorbent article, extending in a longitudinal direction and a transversal direction, wherein the article has a liquid permeable wearer-facing side and liquid impermeable garment-facing side;
- pouring a liquid on the region of interest of the wearer-facing side of the article;
- waiting for the liquid to be absorbed by the absorbent article;
- applying a powder material on the region of interest where the liquid was poured;
- applying a testing device having a contact surface on the region of interest on top of the powder material;
- optionally applying a downward force on the contact surface;
- forcing air to circulate in the direction of the region of interest so that the powder is removed from under the contact surface by the gas flow proportionally to the horizontal permeability of the wearer-facing side;
- visually assessing the amount of powder removed by the air flow.

The method is particularly useful to visualize the high air permeability of articles comprising macroscopic channels on their wearer-facing side. The method may also be used for comparing the horizontal air permeability of the wearer-facing side of a first personal hygiene article and a second hygiene article, by conducting the method simultaneously or subsequently on the first article and the second article.

In a further aspect, the invention can be used to visualize the rewet at the wearer-facing side of the article if the contact surface comprises a material that becomes sticky when wet, such as a filter paper. In that case the method can comprise the further steps of:
- removing the testing device;
- visually assessing the amount of powder that has stick on the contact surface of the testing device.

This and further aspects of the method of the invention are indicated in the claims, and are further described in the following description.

### BRIEF DESCRIPTION OF THE DRAWINGS

Fig. 1 shows the wearer-facing surface of an exemplary article in the form of a diaper which has been attached flat to a hard surface;
Fig. 2 shows a test liquid being applied to a region of interest at the surface of the diaper;
Fig. 3 shows the diaper immediately after the liquid being absorbed with macroscopic channels formed;

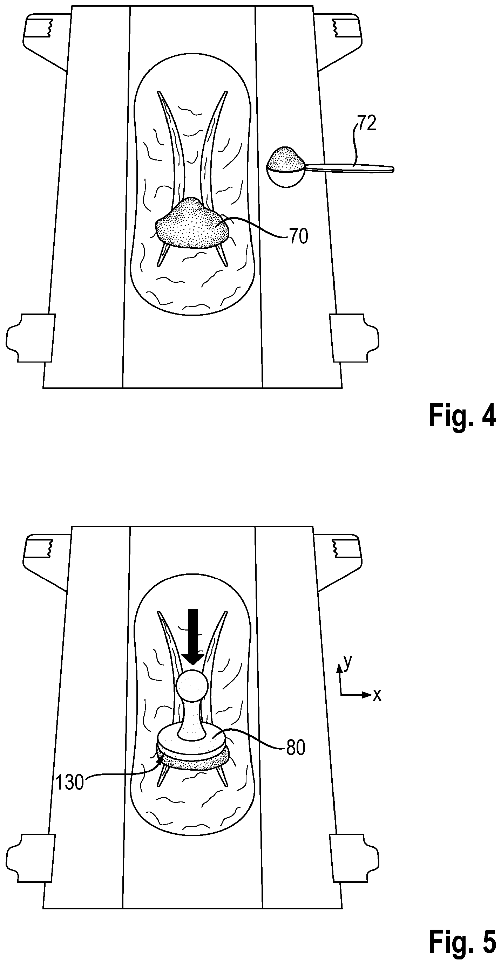

Fig. 4 shows a powder being applied on the surface of interest;
Fig. 5 shows that a testing device having a circular contact surface applied over the powder on the surface of interest;
Fig. 6 shows air being circulated at the interface between the testing device and the surface of the diaper;
Fig. 7 shows the powder residue that remained in the region of interest and at the contact surface of the testing device.

### DETAILED DESCRIPTION OF THE INVENTION

### Introduction

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of' which excludes any element, step or ingredient not mentioned which materially affect the way the feature performs its function, and the term "consisting of' which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

As used herein, the term "wearer" refers to the person, which may be an adult, child, or baby, that suffers from incontinence and actually wears the absorbent product. The term "user" refers to the caregiver that applies the absorbent article on the wearer. The "user" may be a parent, a professional caregiver, or the wearer. The "tester" refers to the person conducting the method of the invention. The wearer, user and tester may be the same or different persons.

The invention will now be further illustrated with reference to the embodiment as described in the Figures with reference to the numerals referred to in these Figures. However it should be understood that this exemplary embodiment and the numerals are not intended to limit the scope of the claims, unless specifically indicated. Dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

### Article to be tested

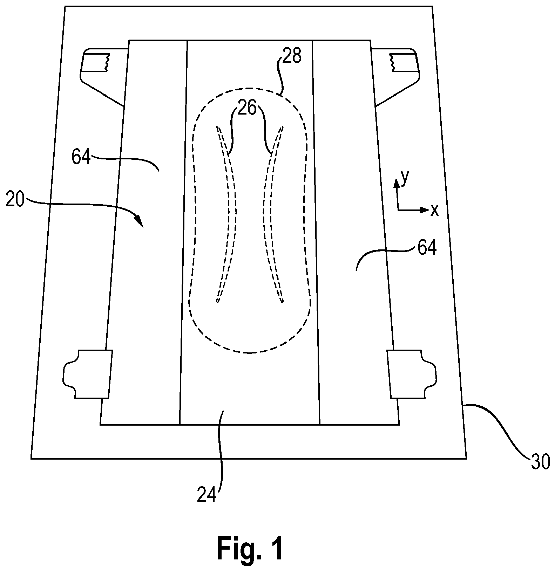

Fig. 1 shows the wearer-facing surface of a diaper 20 which has been attached flat to a hard support surface 30. The support surface may be any kind of hard or rigid surface such as a smooth plastic board made of polycarbonate or acrylic glass, or glass, metal etc.. This surface 30 is not represented in the next Figures for simplicity but the diaper continues to be attached thereto. As used herein, the term "diaper" is used interchangeably with the term "personal absorbent hygiene article", "absorbent article" and the like, and covers in a broad sense any absorbent articles such as taped diapers, pant-type diapers, training pants, adult incontinence pants and feminine sanitary pads that are worn in the crotch region of a wearer to absorb bodily exudates such as urine or menses. The garment-facing side of the diaper can be attached flat to the hard surface using an adhesive such as double-sided sticky bands or clamps (not represented) even in the presence of elasticized components in the diaper. Pant-type diapers, which have pre-formed side seals and can be fitted on a wearer like a textile underpants, can be attached flat on a surface after opening the side seams, as is known in the art.

The wearer-facing surface of the diaper typically comprises a fluid permeable topsheet 24 and barrier leg cuffs 64 that can be elasticized on the longitudinally extending side edges of the diaper. The diaper is of course represented in a very schematic manner in the Figures for simplicity, so that many of the diaper components such as the elastics of the cuffs are omitted. The topsheet 24 typically forms most of the wearer-contacting surface of the article and is typically the first layer that the body exudates contact during wear. The topsheet is a liquid permeable layer that allows liquids to readily penetrate through its thickness. The topsheet is preferably compliant, soft-feeling, and non-irritating to the wearer's skin. The diapers may have any conventional topsheets and suitable topsheets may be manufactured from a wide range of materials. Most topsheets are or comprise nonwoven materials or apertured formed films. The articles also typically comprise cuffs 64 that improve the fit of the article around the legs of the wearer and that are visible on the wearer-facing side of the article. Such cuffs typically comprise barrier leg cuffs (also referred to as inner cuffs) and gasketing cuffs (also referred to as outer cuffs) as is known in the art.

The diaper comprise a garment-facing side which is the side opposite the wearer-facing side and is typically liquid impermeable. The garment-facing side is typically comprised of a plastic film referred to as backsheet. The backsheet film often comprises a thin layer of nonwoven on its external side to provide a nicer feel to the user. In the present method, the garment-facing side of the diaper is disposed in face-to-face relation with the support surface 30 and is not directly visible in the Figures.

The wearer-facing surface of the diaper is generally flat and extend in a transversal direction (x) and a perpendicular longitudinal direction (y), these directions defining a horizontal plane generally parallel to the support surface on which the diaper is disposed. Of course the thickness of the diaper may locally vary due to the presence or absence of the different components of the diaper as well as optional macroscopic channels 26 which may be perceivable in dry and/or wet state at the surface of the diaper, but these thickness variations are much smaller than the other overall dimensions of the diaper in the transversal and longitudinal direction. The diaper also defines a longitudinal axis (not represented), which is an imaginary line at the surface of the diaper parallel to the longitudinal direction y and extending from the middle of the front edge to the middle of the back edge of the diaper and notionally dividing the diaper if two halves.

The absorbent article typically comprises an absorbent core 28 between the topsheet and the backsheet. As used herein, the term "absorbent core" refers to a component used or intended to be used in an absorbent article and which typically comprises an absorbent material enclosed in a core wrap. As used herein, the term "absorbent core" does not include the topsheet, the backsheet and (if present) any acquisition-distribution layer or multilayer system, which is not integral part of the absorbent core. The absorbent core is typically the component of an absorbent article that has the most absorbent capacity of all the components of the absorbent article, and which comprises all, or at least the majority of, superabsorbent polymer (SAP). The absorbent core may consist essentially of, or consist of, the core wrap, the absorbent material and optionally adhesives. The terms "absorbent core" and "core" are herein used interchangeably. The absorbent material may be SAP mixed with cellulosic fibers or may be free of cellulosic fibers.

The absorbent article may comprise an acquisition layer and/or a distribution layer between the topsheet and the absorbent core. These layers allow to quickly acquire the fluid from the topsheet and distribute the fluid over a larger area to better spread the fluid in the direction of the absorbent core. In typical absorbent articles there is one or two of these layers, which are collectively referred herein as acquisition-distribution system, but it is not excluded that the absorbent article does not comprise any acquisition/distribution layer. An example of distribution layer is for example a latex bonded nonwoven and an example of distribution layer is a cross-linked cellulose layer (see for example WO201493323 (Bianchi et al.) for further description), but many other types of materials layers are known and can be used for an acquisition/distribution system.

The absorbent core and/or the acquisition-distribution system may comprise one, and typically at least a pair, of longitudinally-extending macroscopic channels 26. The macroscopic channels may be formed in these layers by any known means, such as compression if the layer is made of a compressible material, or by providing the layers with areas which have a lower basis weight amount than the rest of the layer in which they constituted. Examples of channels in these layers are exemplified below. The present method is particularly interesting to visually demonstrate the high horizontal air flow permeability of absorbent article with channels, compared to absorbent articles without these channels.

### Channels 26

The absorbent article may comprise at least a pair of channels 26, each channel being disposed on a different side of the longitudinal axis of the article as illustrated in Fig. 1. The longitudinal axis of the article longitudinally divides the absorbent article in a symmetrical right and a left side. It is not excluded that the article may also comprise more than two channels or only one channel (in the following "channels" includes the singular and plural form). The term channel designates an area of a component of the article that comprises less absorbent material or compressed material relative to the surrounding areas of that component. An insulting fluid can be quickly distributed along channels towards the front and back of the article for example. The channels may also serve to facilitate the folding of the article in a desired configuration during wear. For the avoidance of doubt, the term "channels" refers herein to macroscopic structures which are clearly recognizable in the diaper, and which measure at least 5 mm in the longitudinal direction, and does not include micro-channels present for example between fibers in a nonwoven or between grains of SAP.

The channels may be present in the absorbent core 28 and/or any of the layer of an acquisition-distribution system if present. The channels may be substantially free of absorbent material and/or acquisition/distribution material. By "substantially free" it is meant that in each of the channels, the basis weight of the absorbent material or distribution/acquisition material is at least less than 25% (in particular less than 20%, less than 10%) than the average basis weight of the absorbent material in the rest of the layer considered. In particular, channels in the absorbent core may be free of absorbent material. Minimal amount such as involuntary contaminations with absorbent material particles that may occur during the making process are disregarded for the purpose of considering if there is absorbent material in the channels.

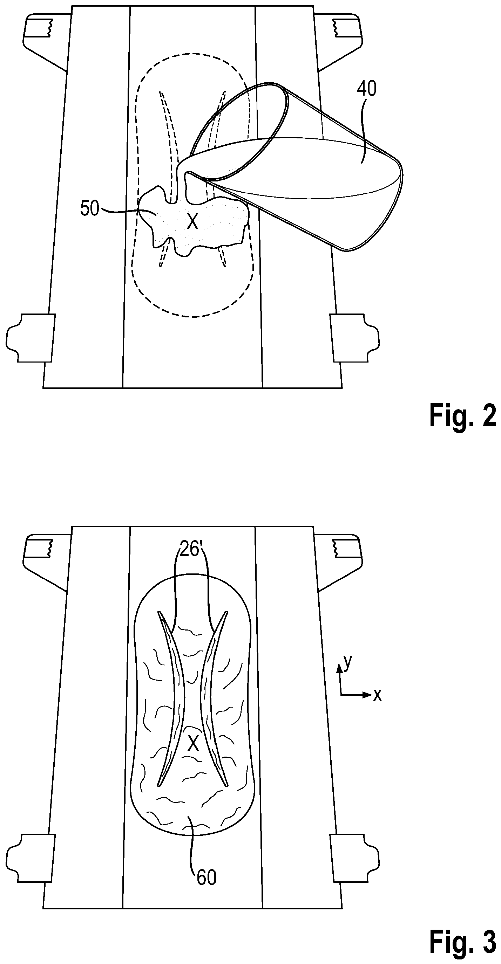

Channels can be typically present in the absorbent core, as for example disclosed in WO2012/170778 (Rosati et al.), WO2014/93311A1 (Arizti et al), WO2014/093310 (Ehrnsperger et al.), which disclose absorbent structures that comprise superabsorbent polymers, optionally a cellulosic material, and at least a pair of substantially longitudinally extending channels. Absorbent cores typically comprise an absorbent material 60 enclosed in a core wrap. The top side and the bottom side of the core wrap may be bonded to each other through these channels by channel bonds, for example by adhesive or other known methods. If the core is sufficiently thin, these channels may not be easily recognizable in the dry state by a user. Channel bonds allow the channels 26 to form more pronounced three-dimensional channels 26' as the absorbent material swells when it absorbs a liquid such as urine as illustrated in Fig. 3. Channels 26 substantially free of absorbent material can be formed for example in a airfelt-free core by modifying the pattern of the grid and receiving drums so that no SAP is applied in selected areas, as exemplary disclosed in US2012/0312491 and US2014/027066 (Jackels et al.). The absorbent core can alternatively comprise channels without core wrap bonds, or may be without channels or channel-forming areas

In addition or alternatively to channels in the core, channels may also be present in the acquisition/ distribution system (that in any of the layers of the system if it is a multi-layer system). Since these intermediate layers are typically placed directly under the topsheet, these channels may be more apparent even before wetting when the diaper is still in the dry state. Such channels in a layer of the distribution-acquisition system are for example disclosed in WO2015/031225 (Roe et al.). The channels in the acquisition/distribution system, if present, can cooperate with the channels present in an absorbent core, when these are at least partially superposed with the channels of the acquisition/distribution system.

The channels typically extend generally longitudinally, which means that each channel extends at least as much in the longitudinal direction (y) than in the transversal direction (x), and typically at least twice as much in the longitudinal direction than in the transverse direction (as measured after projection on the respective axis). The channels 26 may have a length L' projected on the longitudinal axis that is at least 20% of the length L of the absorbent article, in particular from 30% to 85%. The channels may for example have a width Wc along at least part of their length of at least 2 mm, or at least 3 mm or at least 4 mm, up to for example 20 mm, or 16 mm, or 12 mm. The width Wc may be constant through substantially the whole length or may vary along the length of the channels. The channels may be formed by areas substantially free of absorbent material, The channels are advantageously surrounded by the material of the layer in which they are formed, which means that the channels do not extend to any of the edges of the layer.

The channels 26 may be curved towards the longitudinal axis as shown in the Figures, but they may be also straight and parallel to the longitudinal axis. There may be or may be no channels that coincide with the longitudinal axis of the core. When present as a pair of channels disposed symmetrically relative to the longitudinal axis of the core, these may be spaced apart from one another over their whole longitudinal dimension. The smallest spacing distance may be for example at least 5 mm, or at least 10 mm, or at least 16 mm.

The profile (depth) of the channels at the wearer-facing side of the article can be at least 1 mm in the dry state, and can be typically at least 1 mm or more (e.g. at least 2 mm, or at least 3 mm) when the article is wet. In particular, when channels are present in the absorbent core, they will become deeper when the absorbent article is wet due to the swelling of the absorbent material around the channels.

The method of the invention is particularly useful to visualize the horizontal air permeability of diaper comprising channels in at least one internal layers (absorbent core and/or an acquisition layer and/or distribution layer, especially when the channels have a recognizable three-dimensional profile at the wearer-facing the surface of the article in the wet state. However the method can also be used on articles having no channels, for example as comparison to articles having channels.

### Liquid pouring

Fig. 2 illustrates the step of pouring a liquid 40 on a region of interest 50 of the wearer-facing side of the article. The region of interest is the region for which the horizontal air permeability is desirably demonstrated. The region of interest can be for example centered on the typical point of fluid insult for the type of article tested. For a size 4 baby diaper which is about 460 mm long, the region of interest 50 may be for example centered on the longitudinal axis of the diaper at a distance of about 160 mm from the diaper's front edge, as represented by the X cross on the Figures. More generally, the liquid may be typically poured towards the middle or the front of the article. If the article comprises channels, the region of interest may be a region close to at least a portion of the channels. In particular, if a pair of symmetrical channels are present, the liquid may be poured between the pair of channels 26 towards the center or the front of the article.

The amount of liquid to be poured may represent a typical "gush" of liquid exudate for the article considered, which will vary according to the size of the prospective wearer of the article, the severity of the incontinence and the type of exudates. Advantageously, a pre-determined amount of liquid will be poured to ensure reproducibility, especially if two different products are to be tested and compared. The amount of liquid may be pre-determined, for example to simulate a first gush of urine from a baby, a liquid amount of about 75 ml may be used for a (Size 4) diaper (more generally between 50 ml and 100 ml), but for smaller or larger sizes other amount may be used (for example from 20 ml to 200 ml). The tester may alternatively use a relatively imprecise amount of fluid (a small glass of water for example) if only a qualitative comparison is desired. A second amount of liquid may be poured after the first pouring of the liquid to simulate a second gush of urine, for example the same amount as previously poured, or more generally any amount of liquid.

The liquid 40 provided may be any liquid that can be absorbed by the absorbent article. Typically an aqueous liquid will be provided. Tap water may for example be used without any further preparation for the liquid. A more sophisticated liquid may be also prepared, for example comprising a colorant to better highlight the swollen absorbent material. Typical colorants used for demonstration provide water with a blue or green color but any other color may also be used. The test liquid may also comprise some sodium chloride to better simulate urine. The liquid may be contained in any type of container such as a beaker glass ort a small bottle. The container may be advantageously graduated so that the person conducting the test can control in a reproducible manner the amount of fluid dispensed on the article.

After the desired amount of liquid has been applied, a few seconds will be typically needed for the liquid to penetrate through the topsheet inside the article and some more time for the article to absorb the liquid. Typically this can take place in less than 1 minute. If the absorbent core comprises channels 26, as indicated previously, these will typically form more pronounced three-dimensional depressions 26' visible on the wearer-facing surface. This is because the absorbent material 60 surrounding the channel areas swells whereas the channels will not swell or at least swell much less. Channels present in a distribution / acquisition system will be typically already clearly perceivable on the wearer-facing side of the article before adding the liquid.

### Powder material 70

After the liquid 40 has been poured and absorbed by the article, a powder 70 is carefully applied on the zone of interest. The powder can be applied per hand with the fingers, or using a spoon 72 or any other similar implement. Advantageously, a predetermined amount of powder is weighted before being sprinkled on the area of interest to facilitate comparison between two different tested articles. The powder may be any powder which is fine enough to be entrained in an air flow and is safe to use in particular hypoallergenic. An example of such powder is baby powder (talc) which may be colored to provide for a better visualization. For a size 4 baby diaper for example, a teaspoon containing 1.5-1.7 g of talc baby powder may be used, but smaller or larger amount of powder may be used.

The powder is applied as uniformly as possible on an area about the same size as the contact surface that will be applied in the next step. The area of application powder may also be smaller or larger than the contact surface as it is typically difficult to apply the powder to an area exactly matching the contact surface. Alternatively a form such as a cylinder can be used to more exactly circumscribe the powder to a particular area.

### Testing device 80 having a flat contact surface 130

Once the powder has been applied, a testing device 80 having a contact surface 130 can be applied on the region of interest on top of the powder material. The contact surface is preferably flat. The contact surface may have any geometry and in particular it may be disc shaped. For a size 4 diaper for example, the contact surface may be for example a disc having a diameter of 60 mm. The testing device may have a handle to facilitate application and removal on the region of interest. The testing device may be made for example of plastic or metal. If the testing device is relatively light, in an optional step a downward force may be applied uniformly on the testing device to make sure that the device is well balanced and that is as stable as possible on the wearer-contacting surface. This can be done for example by applying a weight (not represented) of for example 500 grams on top of the device. A higher or lower mass is of course possible as long as the downward force does not damage the article and provides for the testing device rest on the region of interest in a stable manner. The weight may be left for a limited amount of time, for example 3 seconds and then removed before conducting the next step, or the weight may be left on the testing device during the next step. It is also possible to manually push the testing device downwards for a limited amount of time (e.g. 3 seconds) if adapted weights are not available.

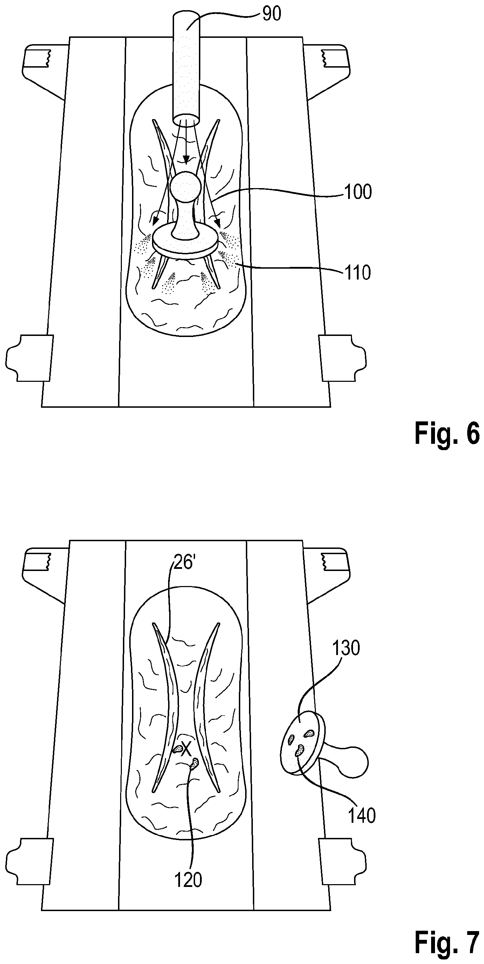

In an optional step, not represented in the Figures, the contact surface 130 of the testing device 80 may be fitted with a substrate that becomes sticky when wet. The substrate that can be attached to the contact surface may be for example standard laboratory filter paper or general purpose tissue paper. This additional step is useful in a further aspect of the invention to visualize the amount of rewet at the surface of the article, as will be discussed in relation to Fig. 7. The substrate may be cut to match the shape of the contact surface and releasably attached thereto using for example double-sided adhesive tape.

### Forced air circulation and assessment

As next step, air is forced in the direction of the region of interest at the interface between the wearer-facing surface of the diaper and the contact surface of the testing device. The forced circulation may be simply provided by the tester blowing air through a straw 90, at a close position (10-20 cm) in the direction of the interface. Other method of circulation may also be used for example simply blowing air through the lips, using compressed air released through a nozzle, or nasal aspirators, syringes, hair dryers etc.. Ambient air has the advantage of being safe and directly available, but it is not excluded that other common gas such as nitrogen may be used instead. The air flow may be for example directed towards the region of interest at an angle of about 10 degrees to 60 degrees relative to the horizontal. The air flow may also be optionally transversally moved during this step from one side of the testing device to the other side (in the transversal direction) to subject the whole of the contact surface to about the same intensity of the air flow during this step.

As illustrated in Fig. 6, the air flow 100 has for effect to remove the previously deposited powder 70 from under the contact surface 130 of the testing device in proportion of the horizontal air permeability of the wearer-facing side at the point of interest. This is immediately visualized by the streak of powder exiting from under the contact surface on the side opposite of the incoming air flow. Wearer-facing surfaces having high horizontal air permeability, such as those having macroscopic channels 26' on their surface, will have a cloud of powder dust 110 caused by the air flow at the surface of the article which is very visible. On the other hand, for less permeable surface, in particular those not having three-dimensional channels at their surface, there may be much less or even no powder removed from under the contact surface of the testing device. The differences of horizontal air permeability, for example between an article having a wearer-facing surface having three-dimensional channels 26' and an article not having those channels, can thus be immediately demonstrated by this method.

In a further optional step, the testing device 80 can be removed and the powder residue 120 on the wearer-facing side of the article which has not been swept away by the air flow can be visually assessed, as shown on Fig. 7. The amount of residual powder 120 that remains on the wearer-facing side inversely correlates with the horizontal air permeability. The amount of residual powder that remains on the contact surface 140 may also be visually assessed to determine the rewet properties at the wearer-facing surface of the diaper, as will detailed hereunder. Optionally the power residue on both surfaces may also be weighted for more exact results reporting.

### Comparative testing

The method of the invention can be repeated with different absorbent articles to qualitatively compare the different horizontal air permeability properties of the different articles. The articles are advantageously tested using the same conditions for each test. The present method is easy to perform by unskilled testers for qualitative testing but can also be used for quantitative testing by professional or trained testers, for example by measuring the exact amount of powder entrained under precise conditions. The method may also be used to demonstrate that the presence of macroscopic channels in an absorbent article provide improved horizontal air permeability relative to other articles without channels. Such a method for comparing a first absorbent article with a second absorbent article can thus include the steps of:
- performing the method described previously on the first absorbent article to obtain a first result;
- performing the same steps of the method indicated above on the second absorbent article to obtain a second result, and
- comparing the first result to the second result.

The first absorbent article may for example comprise a pair of macroscopic channels and the second absorbent article may be devoid of channels, or vice versa. The result of each test may be reported in common words, for example "no", "small", "medium" or "large" amount of powder blown away or according to any other gradation as is commonly used in testing methods (e.g. numeral gradation).

### Rewet visualization

In a further aspect, the invention can be used to visualize the rewet at the wearer-facing side of the tested article. Rewet means the humidity that can surface again at the surface of the diaper after the fluid has been absorbed, for example when a pressure on the diaper is exerted such as by a baby sitting on the diaper. Rewet should be avoided as it raises the humidity on the skin of the wearer when the skin contacts directly the topsheet.

For this additional method step, the contact surface 130 of the testing device should comprise a material that becomes sticky when wet, such as filter paper, a general use absorbing tissue paper, or a sponge or sponge-like material. This material can be releasably attached to the contact surface of the testing device as has been indicated above. In this case the method can comprise the further steps of:
- removing the testing device 80 from the article tested; and
- assessing the amount of powder 140 that has stuck on the contact surface 130 of the testing device.

The assessment may typically be visual and measure qualitatively the area of the contact surface comprising powder and/or the amount of powder stuck thereto. The more powder has stuck to the surface, the higher the rewet of the diaper is because the stickiness of the surface is proportional to the amount of liquid it was subjected to. Quantitative assessment may also be optionally conducted by collecting and weighing the powder residue 140 stuck on the testing device. These further steps may be conducted on two different articles to compare their rewet properties, and the rewet results measured may be qualitatively expressed according to any gradation used in the field of testing (e.g. "no", "small", "medium" or "large" rewet, or numeral gradation etc..).
The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method for visualizing the horizontal air permeability of the wearer-facing side of a personal hygiene article (20) such as a diaper, the method comprising the subsequent steps of:
- providing an absorbent article (20), extending in a longitudinal direction (y) and a transversal direction (x), wherein the article has a liquid permeable wearer-facing side and a garment-facing side;
- pouring a liquid (40) on a region of interest (50) of the wearer-facing side of the article;
- waiting for the liquid to be absorbed by the absorbent article;
- applying a powder (70) on the region of interest where the liquid was poured;
- applying a testing device (80) having a contact surface (130), which is preferably flat, on the region of interest on top of the powder;
- optionally applying a downward force on the testing device;
- forcing an air flow (100) to circulate in the direction of the region of interest so that the powder is removed from under the contact surface of the testing device by the air flow proportionally to the horizontal air permeability of the wearer-facing side at the region of interest;
- visually assessing the amount of powder (110) removed by the airflow.

2. A method according to any of the preceding claims, wherein the liquid is water or water-based and optionally comprises a colorant.

3. A method according to any of the preceding claims, wherein the powder is talc powder.

4. A method according to any of the preceding claims, wherein the downward force is applied on the device for a limited amount of time and is removed before forcing the air flow to circulate.

5. A method according to any of the preceding claims, wherein the steps of pouring the liquid and waiting for the liquid to be absorbed by the absorbent article are repeated at least once.

6. A method according to claim 1, wherein the article comprises a pair of three-dimensional channels (26') at the surface of wearer-facing side and the channels have a depth of at least 1 mm, preferably at least 2 mm, more preferably at least 3mm, after the liquid has been absorbed.

7. A method according to claim 6, wherein the center (x) of the region of interest (50) is between the channels (26').

8. A method according to any claims 6 to 7 wherein the channels (26') are longitudinally extending and the air is forced in the longitudinal direction.

9. A method according to any of the preceding claims 6 to 8, wherein the absorbent article comprises an absorbent core between the wearer-facing side and the garment-facing side, and the absorbent core comprises an absorbent material, and the absorbent core comprises a pair of core channels (26) disposed symmetrically on each side of the longitudinal axis of the article.

10. A method according to claim 9, wherein the absorbent material is comprised in a core wrap having a top side and a bottom side, the core channels (26) are substantially free of absorbent material (60), the top side and the bottom side of the core wrap are bonded to each other through the channels, and the core channels are surrounded by the absorbent material of the core.

11. A method according to any of the claims 6 to 10 wherein the absorbent article comprises a at least one channel in an acquisition layer and/or a distribution layer present under the wearer-facing side of the article.

12. A method according to claim 11, wherein the absorbent article comprises a pair of channels in an acquisition layer and/or a distribution layer present under the wearer-facing side of the article.

13. A method according to any of the preceding claims, wherein the contact surface of the device becomes sticky when wet, and the method further comprises the steps of:
- removing the testing device from the wearer-contacting surface;
- assessing the amount of powder (140) that has stuck on the contact surface (130) of the device to derive therefrom an assessment of the rewet properties of the article tested.

14. A method for comparing the horizontal air permeability of the wearer-facing side of a first personal hygiene article with a second hygiene article, the method including the steps of:
- performing the method of claim 1 on the first absorbent article to obtain a first result;
- performing the method of claim 1 on the second absorbent article to obtain a second result; wherein both steps can be performed simultaneously or in any order; and
- comparing the first result and the second result.

15. A method according to the preceding claim, wherein the first article comprises channels as in any claims 6 to 12, and the second article does not comprise channels.

## Patentansprüche

1. Verfahren zur Sichtbarmachung der horizontalen Luftdurchlässigkeit der trägerseitigen Seite eines Körperhygieneartikels (20), wie einer Windel, wobei das Verfahren folgende Schritte umfasst:
- das Bereitstellen eines Absorptionsartikels (20), der sich in einer Längsrichtung (y) und einer Querrichtung (x) erstreckt, wobei der Artikel eine flüssigkeitsdurchlässige trägerseitige Seite und eine bekleidungsseitige Seite aufweist;
- das Gießen einer Flüssigkeit (40) auf einen Bereich (50) von Interesse der trägerseitigen Seite des Artikels;
- das Abwarten, dass die Flüssigkeit von dem Absorptionsartikel absorbiert wird;
- das Aufbringen eines Pulvers (70) auf den Bereich von Interesse, auf den die Flüssigkeit gegossen wurde;
- das Aufbringen einer Prüfvorrichtung (80) mit einer Kontaktfläche (130), die vorzugsweise flach ist, auf den Bereich von Interesse auf das Pulver;
- wahlweise das Aufbringen einer nach unten gerichteten Kraft auf die Prüfvorrichtung;
- das Drücken eines Luftstroms (100) in eine Zirkulation in Richtung des Bereichs, so dass das Pulver von unterhalb der Kontaktfläche der Prüfvorrichtung durch den Luftstrom entfernt wird, der proportional zu der horizontalen Luftdurchlässigkeit der trägerseitigen Seite in dem interessierenden Bereich verläuft;
- das visuelle Bewerten der Pulvermenge (110), die durch den Luftstrom entfernt wurde.

2. Verfahren nach einem der vorstehenden Ansprüche, wobei die Flüssigkeit Wasser ist oder auf Wasser basiert und wahlweise ein Farbmittel umfasst.

3. Verfahren nach einem der vorstehenden Ansprüche, wobei das Pulver Talkpulver ist.

4. Verfahren nach einem der vorstehenden Ansprüche, wobei die nach unten gerichtete Kraft auf die Vorrichtung über eine begrenzte Zeit hinweg aufgebracht wird und vor dem Drücken des Luftstroms in eine Zirkulation entfernt wird.

5. Verfahren nach einem der vorstehenden Ansprüche, wobei die Schritte des Gießens der Flüssigkeit und des Wartens darauf, dass die Flüssigkeit von dem Absorptionsartikel absorbiert wird, mindestens einmal wiederholt werden.

6. Verfahren nach Anspruch 1, wobei der Artikel ein Paar von dreidimensionalen Kanälen (26') an der Oberfläche der trägerseitigen Seite umfasst und die Kanäle eine Tiefe von mindestens 1 mm, vorzugsweise mindestens 2 mm, mehr bevorzugt mindestens 3 mm aufweisen, nachdem die Flüssigkeit absorbiert worden ist.

7. Verfahren nach Anspruch 6, wobei die Mitte (x) des Bereichs (50) von Interesse zwischen den Kanälen (26') liegt.

8. Verfahren nach einem der Ansprüche 6 bis 7, wobei sich die Kanäle (26') in Längsrichtung erstrecken und die Luft in Längsrichtung gedrückt wird.

9. Verfahren nach einem der vorstehenden Ansprüche 6 bis 8, wobei der Absorptionsartikel einen Absorptionskern zwischen der trägerseitigen Seite und der bekleidungsseitigen Seite umfasst und der Absorptionskern ein Absorptionsmaterial umfasst und der Absorptionskern ein Paar von Kernkanälen (26) umfasst, die symmetrisch auf jeder Seite der Längsachse des Artikels angeordnet sind.

10. Verfahren nach Anspruch 9, wobei das Absorptionsmaterial in einer Kernumwicklung enthalten ist, die eine Oberseite und eine Unterseite aufweist, wobei die Kernkanäle (26) im Wesentlichen frei von Absorptionsmaterial (60) sind, wobei die Oberseite und die Unterseite der Kernumwicklung miteinander durch die Kanäle verbunden sind und die Kernkanäle von dem Absorptionsmaterial des Kerns umgeben sind.

11. Verfahren nach einem der Ansprüche 6 bis 10, wobei der Absorptionsartikel mindestens einen Kanal in einer Aufnahmeschicht und/oder einer Verteilungsschicht umfasst, die unter der trägerseitigen Seite des Artikels vorhanden ist.

12. Verfahren nach Anspruch 11, wobei der Absorptionsartikel ein Paar von Kanälen in einer Aufnahmeschicht und/oder einer Verteilungsschicht umfasst, die unter der trägerseitigen Seite des Artikels vorhanden ist.

13. Verfahren nach einem der vorstehenden Ansprüche, wobei die Kontaktoberfläche der Vorrichtung bei Feuchtwerden klebrig wird und das Verfahren ferner die folgenden Schritte umfasst:
- das Entfernen der Prüfvorrichtung von der trägerkontaktierenden Oberfläche;
- das Bewerten der Pulvermenge (140), die auf der Kontaktfläche (130) der Vorrichtung klebt, um daraus eine Bewertung der Wiederbefeuchtungs-Eigenschaften des geprüften Artikels abzuleiten.

14. Verfahren zum Vergleichen der horizontalen Luftdurchlässigkeit der trägerseitigen Seite eines ersten Artikels für die Körperhygiene mit einem zweiten Artikel für die Körperhygiene, wobei das Verfahren die folgenden Schritte einschließt:
- das Durchführen des Verfahrens nach Anspruch 1 auf dem ersten Absorptionsartikel, um ein erstes Ergebnis zu erhalten;
- das Durchführen des Verfahrens nach Anspruch 1 auf dem zweiten Absorptionsartikel, um ein zweites Ergebnis zu erhalten;
wobei beide Schritte gleichzeitig oder in beliebiger Reihenfolge durchgeführt werden können; und
- das Vergleichen des ersten Ergebnisses mit dem zweiten Ergebnis.

15. Verfahren nach dem vorstehenden Anspruch, wobei der erste Artikel Kanäle wie in den Ansprüchen 6 bis 12 umfasst und der zweite Artikel keine Kanäle umfasst.

## Revendications

1. Procédé pour visualiser la perméabilité à l'air horizontale du côté faisant face au porteur d'un article d'hygiène personnelle (20) tel qu'une couche, le procédé comprenant les étapes suivantes consistant à :
- fournir un article absorbant (20), s'étendant dans une direction longitudinale (y) et une direction transversale (x), l'article ayant un côté faisant face au porteur perméable aux liquides et un côté faisant face au vêtement ;
- verser un liquide (40) sur une région d'intérêt (50) du côté faisant face au porteur de l'article ;
- attendre que le liquide soit absorbé par l'article absorbant ;
- appliquer une poudre (70) sur la région d'intérêt où le liquide a été versé ;
- appliquer un dispositif de test (80) ayant une surface de contact (130), qui est de préférence plate, sur la région d'intérêt par-dessus la poudre ;
- appliquer facultativement une force vers le bas sur le dispositif de test ;
- forcer un flux d'air (100) à circuler dans la direction de la région d'intérêt de sorte que la poudre soit retirée de sous la surface de contact du dispositif de test par le flux d'air proportionnellement à la perméabilité à l'air horizontale du côté faisant face au porteur au niveau de la région d'intérêt ;
- évaluer visuellement la quantité de poudre (110) retirée par le flux d'air.

2. Procédé selon l'une quelconque des revendications précédentes, dans lequel le liquide est de l'eau ou à base d'eau et comprend facultativement un colorant.

3. Procédé selon l'une quelconque des revendications précédentes, dans lequel la poudre est de la poudre de talc.

4. Procédé selon l'une quelconque des revendications précédentes, dans lequel la force vers le bas est appliquée sur le dispositif pendant une durée limitée et est retirée avant de forcer le flux d'air à circuler.

5. Procédé selon l'une quelconque des revendications précédentes, dans lequel les étapes consistant à verser le liquide et à attendre que le liquide soit absorbé par l'article absorbant sont répétées au moins une fois.

6. Procédé selon la revendication 1, dans lequel l'article comprend une paire de canaux tridimensionnels (26') au niveau de la surface du côté faisant face au porteur et les canaux ont une profondeur d'au moins 1 mm, de préférence d'au moins 2 mm, plus préférablement d'au moins 3 mm, après que le liquide a été absorbé.

7. Procédé selon la revendication 6, dans lequel le centre (x) de la région d'intérêt (50) se trouve entre les canaux (26').

8. Procédé selon l'une quelconque des revendications 6 à 7 dans lequel les canaux (26') s'étendent longitudinalement et l'air est forcé dans la direction longitudinale.

9. Procédé selon l'une quelconque des revendications précédentes 6 à 8, dans lequel l'article absorbant comprend une âme absorbante entre le côté faisant face au porteur et le côté faisant face au vêtement, et l'âme absorbante comprend un matériau absorbant, et l'âme absorbante comprend une paire de canaux d'âme (26) disposés symétriquement sur chaque côté de l'axe longitudinal de l'article.

10. Procédé selon la revendication 9, dans lequel le matériau absorbant est compris dans une enveloppe d'âme ayant un côté supérieur et un côté inférieur, les canaux d'âme (26) sont sensiblement exempts de matériau absorbant (60), le côté supérieur et le côté inférieur de l'enveloppe d'âme sont liés l'un à l'autre par les canaux et les canaux d'âme sont entourés par le matériau absorbant de l'âme.

11. Procédé selon l'une quelconque des revendications 6 à 10 dans lequel l'article absorbant comprend au moins un canal dans une couche d'acquisition et/ou une couche de distribution présentes sous le côté faisant face au porteur de l'article.

12. Procédé selon la revendication 11, dans lequel l'article absorbant comprend une paire de canaux dans une couche d'acquisition et/ou une couche de distribution présentes sous le côté faisant face au porteur de l'article.

13. Procédé selon l'une quelconque des revendications précédentes, dans lequel la surface de contact du dispositif devient collante lorsqu'elle est mouillée, et le procédé comprend en outre les étapes consistant à :
- retirer le dispositif de test de la surface en contact avec le porteur ;
- évaluer la quantité de poudre (140) qui a collé sur la surface de contact (130) du dispositif pour en déduire une évaluation des propriétés de remouillage de l'article testé.

14. Procédé de comparaison de la perméabilité à l'air horizontale du côté faisant face au porteur d'un premier article d'hygiène personnelle avec un deuxième article d'hygiène, le procédé incluant les étapes consistant à :
- effectuer le procédé selon la revendication 1 sur le premier article absorbant pour obtenir un premier résultat ;
- effectuer le procédé selon la revendication 1 sur le deuxième article absorbant pour obtenir un deuxième résultat ;
les deux étapes pouvant être effectuées simultanément ou dans n'importe quel ordre ; et
- comparer le premier résultat et le deuxième résultat.

15. Procédé selon la revendication précédente, dans lequel le premier article comprend des canaux tels que dans l'une quelconque des revendications 6 à 12, et le deuxième article ne comprend pas de canaux.
